(19)

Europäisches Patentamt

European Patent Office

Office européen des brevets

(11) **EP 1 979 479 B1**

(12) **EUROPEAN PATENT SPECIFICATION**

(45) Date of publication and mention of the grant of the patent:
**23.09.2015 Bulletin 2015/39**

(21) Application number: **07700018.0**

(22) Date of filing: **03.01.2007**

(51) Int Cl.:
***C12N 13/00*** (2006.01)

(86) International application number:
**PCT/IB2007/000044**

(87) International publication number:
**WO 2007/077532 (12.07.2007 Gazette 2007/28)**

(54) **METHOD FOR MAKING ENDOGENOUS IONS AVAILABLE AND APPARATUS ABLE TO IMPLEMENT THIS METHOD**

VERFAHREN, UM ENDOGENE IONEN VERFÜGBAR ZU MACHEN, UND APPARAT, DER ZUR DURCHFÜHRUNG DIESES VERFAHRENS GEEIGNET IST

PROCÉDÉ DE MISE A DISPOSITION D'IONS ENDOGÈNES ET APPAREIL CAPABLE DE METTRE EN OEUVRE CE PROCÉDÉ

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.01.2006 IT PD20060004**

(43) Date of publication of application:
**15.10.2008 Bulletin 2008/42**

(73) Proprietor: **Prometeo S.r.l.**
**35142 Padova (IT)**

(72) Inventor: **TALPO, Getullio**
**deceased (IT)**

(74) Representative: **Gallo, Luca et al**
**Gallo & Partners S.r.l.**
**Via Rezzonico, 6**
**35131 Padova (IT)**

(56) References cited:
**EP-A1- 0 407 006    WO-A-00/76582**
**US-A- 4 818 697**

- **SMITH STEPHEN D ET AL: "Testing the ion cyclotron resonance theory of electromagnetic field interaction with odd and even harmonic tuning for cations" BIOELECTROCHEMISTRY AND BIOENERGETICS, vol. 38, no. 1, 1995, pages 161-167, XP002435026 ISSN: 0302-4598**
- **THOMAS J R ET AL: "LOW-INTENSITY MAGNETIC FIELDS ALTER OPERANT BEHAVIOR IN RATS" BIOELECTROMAGNETICS, vol. 7, no. 4, 1986, pages 349-358, XP002435027 ISSN: 0197-8462**

EP 1 979 479 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to an apparatus for making endogenous ions physiologically available in an organism.

For some time the effects of ELF (extremely low frequency) magnetic fields both on biological, animal and plant systems and on non-biological systems (for example, aqueous solutions of ions) have been studied. In particular attention has been concentrated on the effects of low-intensity and low-frequency magnetic fields superimposed on a static magnetic field.

In this context it is necessary to mention the patent US 4818697 relating to a technique for increasing the permeability of ions through membranes, in particular cellular membranes, using ELF magnetic fields. This technique envisages increasing the permeability of a given ion through a membrane, which is subject to the earth's static magnetic field or to any other static magnetic field which is arbitrarily chosen, superimposing on this static magnetic field a variable magnetic field modulated with a frequency F proportional to the ratio $Q/m$ of charge Q to mass $m$ of the ion in accordance with the relation which defines the cyclotron frequency $F_c$, namely

$$2\pi \; F_c = \; Q/m \; B_0$$

where $Q$ and m are the charge and the mass of the ion expressed respectively in Coulombs and in kg, and $B_0$ is the intensity of the static magnetic field expressed in Tesla. This technique, which envisages the use of Helmholtz coils for the generation of the variable magnetic field, is claimed to allow regulation, at a speed and with a degree of precision hitherto unknown, of the ion exchange between the outside and the inside of a cellular body.

Recently in the wake of this growing interest in the subject, important progress has been made in understanding the physical phenomena underlying the interaction between ELF magnetic fields and biological and non-biological systems, as testified by numerous scientific articles published on the subject during the last few years.

An important scientific article is that written by E. Del Giudice, M. Fleischmann, G. Preparata and G. Talpo, "Gli irragionevoli effetti dei campi magnetici ELF su un sistema di ioni"["The irrational effects of ELF magnetic fields on an ion system"] published by Bioelectromagnetics 23:522-530 (2002), and incorporated here by way of reference and in support of the scientific claims forming the basis of the present invention.

In greater detail, in the article written by *Del Giudice* et *al.* reference is made to an experiment carried out on a non-biological system of ions at room temperature, in which it is shown how it is possible to induce microscopic ionic discharges of a particular ion species by simultaneously applying two parallel magnetic fields, one of which is static and fairly weak $B_0$ and the other one alternating and much weaker $B_c$, where $B_c \sim 10^{-3} B_0$, the frequency of which coincides with the cyclotron frequency of the chosen ion species. During the experiment ion discharges lasting up to 20 s and with an amplitude of up to 10 nA were observed and it was noted that the much greater energy exchanges induced by thermal agitation did not appear to play any role (so-called "kT problem").

The authors of this article have analysed the problem within the context of quantum electrodynamics, reaching the following conclusions:

- as already demonstrated in previous articles, the molecules of water in the liquid and the ions in the solution are involved, in their basic state, in coherent ordered configurations, called coherence dominions CD;
- the ions are able to move without colliding with each other in spatial environments concentric with the abovementioned coherence dominions of the water;
- owing to the coherence, in these environments the ions are able to follow conventional orbits coaxial with the static magnetic fields.

Introducing for the sake of simplicity some conceptual simplifications, according to abovementioned article, the interstitial spaces which surround the coherence dominions of the water and which, below, for the sake of simplicity will be referred to by the abbreviation CDF (Coherence Dominion Frontier), are well-defined regions of space which surround each coherence dominion CD. More precisely a CDF is the space included between the edge of the CD, identifiable as a sphere with a radius of 500 A°, and a concentric sphere with a radius of 750 A°. Within this space molecular collisions are practically zero (entropy practically zero or kT=0). Only the positive ions dissolved in the water are able to enter a CDF by means of diffusive processes. In view of the absence of molecular collisions in a CDF, even a weak static magnetic field $B_0$ such as the earth's magnetic field is able to induce rotation of the ions in circular orbits with an angular frequency equal to the cyclotron frequency $F_c$ of each ion species.

The positive ions trapped in a CDF are forced during their circular movement to follow orbits, the radius of which is defined by the composition of two opposing forces: one, which is typical of all coherent sets, called a ponderomotive force, which tends to attract the ions towards the CD (or the smaller-radius sphere) with a force proportional to its mass, and the other of the Coulomb type, generated by the potential of an electronic cloud located on the outer edge of the CDF (or the larger-radius sphere). The potential of this electronic cloud does not have effects outside of the CDF according to that which can be established from other articles already published on this subject.

If the ions rotating in a CDF with a cyclotron frequency $F_c$ are subject to a variable magnetic field $B_c$, parallel to the static field $B_0$ and with an effective intensity which is non-zero, having the same cyclotron frequency $F_c$ or the frequency of its harmonics or subharmonics, then these ions are subject to Langvin precession and leave the CDF.

For some time now, in the medical and scientific field, the fundamental role played by many ions in various physiological processes and in particular in the processes for activating certain enzymatic reactions has been firmly established.

Of particular importance is the role played by some metal ions, for example $Li^+$, $Mg^{2+}$, $Ca^{2+}$, $Na^+$, $K^+$, $Cu^{2+}$, $Zn^{2+}$, $Fe^{2+}$ and $Fe^{3+}$, in the activation of enzymatic reactions based on metal enzymes (i.e. with metal ions incorporated in the molecular structure, such as for example, iron in myosin or in haemoglobin) or on metal-activated enzymes (i.e. enzymes capable of interacting with specific ions, for example calsequestrin with calcium).

At the present time it is difficult, if not impossible, to influence in an effective manner physiological reactions and in particular reactions of the enzymatic type.

The attempts to stimulate some of these reactions by modifying the quantity, present in the organism, of the ions associated with them has not produced significant results.

It has been established, in fact, that an increase in the quantity of ions present inside an organism, achieved for example by means of exogenous administration, does not necessarily result in an increase in the quantity of the said ions actually available for the abovementioned reactions. In fact, one fraction of the ions administered is immediately trapped in the CDF of the water present in the organism, while the remaining fraction is involved in a series of complex metabolic reactions, at the end of which the ions involved may be combined in chemical compounds so as to be no longer physiologically available.

It must also be remembered that enzymatic reactions require well-defined activation energies (known as conformational energies) which are supplied locally by the organism and which at the moment cannot be provided from outside in the appropriate amounts and at the appropriate times.

The article "Testing the ion cyclotron resonance theory of electromagnetic field interaction with odd and even harmonic tuning for cations" of Smith et al. reports the result of a study conducted exposing seeds to combined parallel static and sinusoidal magnetic fields turned to the cyclotron resonance harmonics for calcium and potassium ions.

The article "Low-intensity Magnetic Fields Alter Operant Behavior in Rats" of Thomas et al. discloses a study on the behavior of rats affected by the combination of a static magnetic field and a variable magnetic field imposed on them. The above mentioned article describes in particular an apparatus for imposing the static and variable magnetic fields which comprises a magnetometer able to detect the local geomagnetic field and means for generating a variable magnetic field which comprise Helmholtz coils.

It is known from WO 00/07664 an apparatus for treating the human body with variable magnetic fields which comprises a waveform generator to be connected to Helmholtz coils which produce low-intensity, low-frequency variable magnetic fields. The apparatus further comprises an impedance meter to be applied to the person to evaluate the effectiveness of the waves of the generator.

Document US 4,818,697 discloses a method for enhancing the transport of a selected ion through a cell membrane which envisage to apply on the cell membrane a variable magnetic field.

It is moreover known from patent application WO 00/76582 an apparatus for ion transport activation through cell membranes of living organism, which comprises waveform generation means connected to a variable magnetic field generation means containing at least one electromagnetic coil. The waveform generation means are able to generate a signal deriving from the combination of two or more waves.

Document EP 0407006 discloses an apparatus for regulating the movement of selected ion accross a membrane, which comprises waveform generation means connected to means for generating an applied variable magnetic field having one or more Helmholtz coils.

In this situation, therefore, one object of the present invention is to overcome the drawbacks of the prior art mentioned by providing an apparatus for making endogenous ions physiologically available inside the cells of an organism without modifying the quantity of these ions present in the organism itself.

A further object of the present invention is to provide an apparatus for making endogenous ions physiologically available which is able to provide jointly the energies which place the organism in a condition for activating specific physiological reactions inside or outside the cells.

A further object of the present invention is to provide an apparatus which is inexpensive from a cost point of view and is operationally entirely reliable.

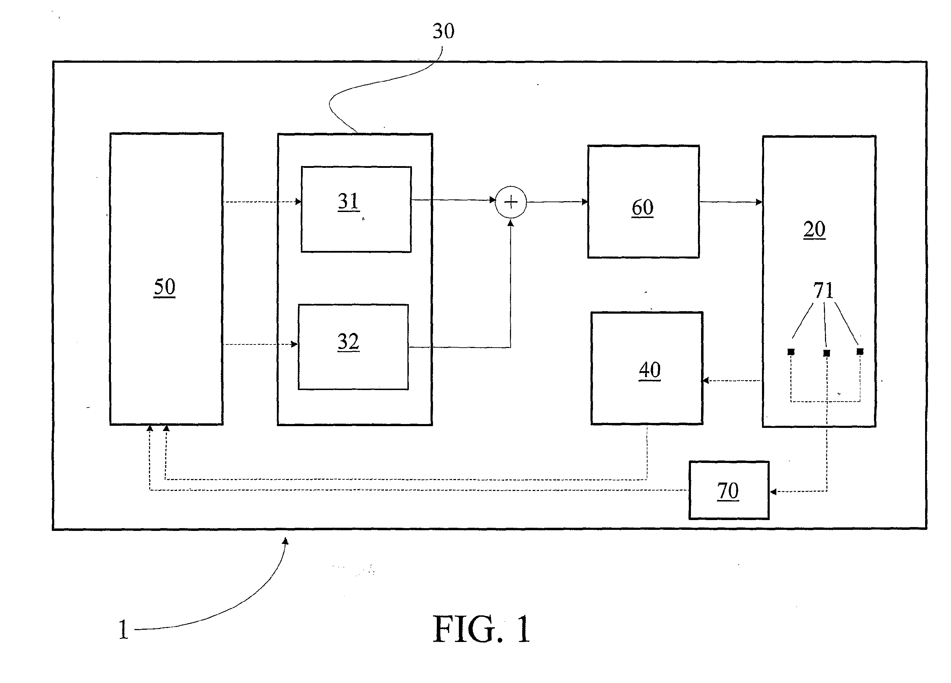

The technical features of the invention, according to the abovementioned objects, may be clearly determined from the accompanying claims and the advantages thereof will become clear from the detailed description of a purely exemplary and non-limiting embodiment, provided with reference to Figure 1 which shows a block diagram of the apparatus according to a preferred embodiment of the invention.

Detailed description

The apparatus for making endogenous ions physiologically available in an organism, according to the present invention, envisages irradiating animal or plant organisms with ULF (ultra low frequency) or ELF (extremely low frequency) electromagnetic fields whose frequency and intensity are suitably modulated.

The expression "ULF and ELF electromagnetic fields" must be understood as referring here to low-intensity fields (between $10^{-8}$ and $10^{-4}$ Tesla) which have a very low frequency (0 to 3 Hz) and low frequency (3 Hz to 1 kHz), respectively.

Using the apparatus according to the invention it is possible to stimulate in an (animal or plant) organism physical and chemical conditions prior to possible subsequent activation of particular physiological reactions, in an entirely non-invasive manner and without the aid of chemical products (drugs, additives, etc.).

In animal organisms, it is possible in particular to induce conditions prior to the start of enzymatic reactions which envisage the involvement of metal ions.

In greater detail, with reference to the principles of quantum electrodynamics already mentioned, the apparatus according to the invention is able to make physiologically available within an organism endogenous ions (i.e. ions already present inside the organism), which otherwise cannot be used by the organism in that they are trapped in the so-called CDF of the intracellular and/or extracellular water.

The ions trapped in the CDF have not been involved in the normal metabolic reactions of the organism and are therefore in an uncombined state which makes them potentially available to act directly in intercellular and/or extracellular physiological reactions, in particular of the enzymatic type.

The term "intracellular" is understood as expressly referring to the inside of the cells, while the term "extracellular" is understood as referring generally to the outside of the cells.

According to the invention, the apparatus allows to accomplish the following steps:

(a) a step for selecting an ion species to be made physiologically available in an organism;
(b) a step for generating a variable ELF or ULF electromagnetic field $B_c$ for irradiating an organism;
(c) a step for detecting the intensity of the local static magnetic field $B_0$ at least in its component parallel to the electromagnetic field $B_c$; and
(d) a step for modulating the main wave which defines the variable electromagnetic field $B_c$ by means of superimposition of a secondary wave having a frequency in the range of 1 to 100 kHz.

During the selection step (a), the ion species to be made physiologically available is chosen depending on the specific physiological reactions which are to be promoted in an organism. As will be described further below, a database which collects the information currently available on the subject has been devised.

For the purposes of the present invention, each ion species is defined by the charge/mass ratio Q/m, expressed in Coulombs/kg. For example, the ions $Ca^{2+}$, $Li^+$, $Na^+$ and $Mg^{2+}$ are defined with a ratio Q/m equal to 4.81 $10^6$ C/kg, 13.9 $10^6$ C/kg, 4.19 $10^6$ C/kg e 7.93 $10^6$ C/kg, respectively.

The detection of the intensity of the local static magnetic field $B_0$ may be performed prior to the abovementioned generation and modulation steps (b), (d) or, alternatively, during these steps continuously or at regular time intervals.

"Local static magnetic field" is understood as meaning here the static magnetic field which is normally present in all environments and may be regarded as constant over time and is defined essentially by the earth's magnetic field (~ 2- 5 $10^{-5}$ Tesla) or by altered levels of the latter owing to the presence of magnetic bodies.

In particular, as will be clarified below, for the purposes of the invention it is important to know the value of the local magnetic field $B_0$ in its component parallel to the variable electromagnetic field $B_c$, in the space where the organism is irradiated with this variable electromagnetic field $B_c$.

Below for the sake of simplicity, "local static magnetic field $B_0$" will be understood as meaning its component parallel to the variable magnetic field $B_c$.

In operationally terms, once the ion species to be made physiologically available has been determined and the intensity of the local static magnetic field $B_0$ is known, the organism is irradiated with the variable electromagnetic field $B_c$ generated during the abovementioned generation step (b).

This field is defined at least by one main wave having a frequency the same as the cyclotron frequency $F_c$ of the chosen ion species or the harmonic or subharmonic frequency of this frequency according to the Bessel series. In this way, in accordance with the initial premises of quantum electrodynamics, the variable magnetic field $B_c$ is able to interact by means of resonance with the ions of the chosen ion species trapped in the CDF of the water of organism, thus causing expulsion thereof.

By way of example, the cyclotron frequencies $F_c$ for the ions $Ca^{2+}$, $Li^+$, $Na^+$ and $Mg^{2+}$, expressed in Hz and calculated using the already mentioned expression $2\pi F_c = Q/m\,B_0$, are equal respectively to the following values 0.79459, 2.29413, 0.69264 and 1.31031 multiplied by the value of the component of the local static magnetic field $B_0$ parallel to the variable electromagnetic field $B_c$ expressed in Tesla.

Preferably, the main wave is of the pulse type and has a non-zero average intensity. It has been established that this favours expulsion of the ions from the CDF.

As will be clarified below, the intensity of the variable electromagnetic field $B_c$ may also be suitably modulated within the range of $10^{-8}$ to $10^{-4}$ Tesla.

During the modulation step (d), the main wave which defines the variable electromagnetic field $B_c$ is superimposed by a secondary wave having a frequency within the range of 1 to 100 kHz. The secondary wave is superimposed on the main wave at time intervals where the latter is not zero.

In this way, the variable electromagnetic field $B_c$ irradiated onto the organism has the properties both of the main wave and of the secondary wave, and in particular the two different frequencies.

The abovementioned modulation of the main wave is a fundamental step in the method according to the present invention in that it provides the variable electromagnetic field $B_c$ with the capacity to penetrate inside the cells of the organism and therefore interact with the ions trapped in the CDF of the intracellular water.

As has been demonstrated by known biophysics studies, the cellular membranes of any organism may be compared, from the point of view of their electrical behaviour, to circuits comprising capacitive, resistive and inductive components combined in series and/or in parallel with each other, in arrangements which vary depending on the type of cell. The presence, in these electrical arrangements, of inductive components combined with capacitive components gives rise to the presence of well-defined resonance frequencies at which the cellular membranes offer the minimum resistance to the passage of variable electromagnet fields.

The Applicant has established that many cell species have resonance frequencies within the range of 1 kHz to 100 kHz. In this context, therefore, the abovementioned step of modulation of the main wave which defines the variable electromagnetic field $B_c$ is of fundamental importance. In fact, in the absence of this second modulation, the variable electromagnetic field $B_c$ would be screened by the cellular membranes and could interact solely with the ions contained in the CDF of the extracellular water.

With the present invention it is therefore possible to make physiologically available in an organism not only the ions trapped in the CDF of the extracellular water, but also the ions trapped in the CDF of the extracellular water, and therefore place the organism in a condition to activate possible subsequent intracellular metabolic reactions which involve these ions.

Advantageously, as will become clear from the continuation of the description, with the apparatus according to the invention it is possible to make available within an organism quantities of energy which are well-defined and in particular can be regulated such that they can be used by the organism itself to activate possible successive physiological reactions which involve the ions expelled from the CDF. These well-defined quantities of energy are provided by electromagnetic fields of the MASER type generated by the expulsion of the ions from the CDF.

In fact, on the basis of theoretical and experimental studies carried out by the inventor, which have been able to develop further the results of *Del Giudice et al.* and add to the knowledge about the physical phenomena which occur in the coherence dominion frontiers (CDF), following expulsion from a CDF of ions contained therein, the electrons orbiting in the external periphery of the CDF are no longer subject to the Coulomb field of the ion species expelled from the CDF and therefore they change orbit, producing an electromagnetic field which may be measured.

This electromagnetic field is a coherent MASER field, the frequency of which depends on the ratio Q/m of charge Q to mass m of the ion species released from the CDF and the intensity of which depends on the charge Q of the ion species released, on the mass m of the ion species released and on the ratio $B_c/B_0$ between the average intensity of the variable electromagnetic field $B_c$ and the intensity of the static electromagnetic field $B_0$ (again understood in terms of its component parallel to the variable electromagnetic field $B_c$).

The power of the abovementioned coherent electromagnetic MASER field emitted by a CDF is defined by the formula:

$$P = Ni\left[\frac{Q^4\,B_0{}^2\,(\omega R)^2}{6\pi\varepsilon°{}^2\,c^3\,mr}\right]$$

where: N stands for the number of ions emitted from the CDF; m = mass of the ion emitted; $\omega$ angular velocity in the cyclotron orbit; R = radius of the CDF (about 750 A°); Q charge of the ion emitted; $B_0$ value of the local static magnetic field in the component parallel to the electromagnetic field $B_c$; mr ratio between the mass of the ion and the mass of the electron; $\varepsilon°$ dielectric constant of the vacuum; $6\pi$ solid output angle of the ion emitted; c speed of light.

For this purpose, in accordance with a preferred solution of the invention, the present apparatus envisages to set the process inputs by following two further steps, that are:

> (e) a step for selecting the intensity of these MASER fields; and
> (f) a step for amplitude regulation of the variable electromagnetic field $B_c$.

Selection of the intensity of the MASER fields emitted is performed depending on the type of physiological reactions to be stimulated, with the aid of the knowledge from specific medical and scientific studies on the subject. On the basis of these studies it is possible to establish for each physiological reaction the ion species associated with it and the quantities of energy which must be made available in an organism in order to make this reaction possible. As already mentioned previously, on the basis of these studies, it has been possible to device a database which associates with each physiological reaction the ion species to be made available (with associated values of the ratio Q/m) and the values of the ratio $B_c/B_0$ corresponding to MASER fields with a specific intensity for the said reaction.

In fact, the intensity of the MASER fields emitted with the expulsion of the ions from the CDF is defined by the value of the ratio $B_c/B_o$ between the average intensity of the variable electromagnetic field $B_c$ and the intensity of the static electromagnetic field $B_0$, as well as by the charge Q and on the mass m of the ion species emitted.

In operational terms, after selecting the intensity of the MASER field which is to be emitted and determining therefore the corresponding value of the ratio $B_c/B_0$, the step (f) of regulating the amplitude of the variable electromagnetic field $B_c$ is performed.

During this step, the average intensity of the field $B_c$ is regulated depending on the value detected for the intensity of the local static magnetic field $B_0$ so as to obtain the predetermined value $B_c/B_0$, by suitably defining the amplitude of the main wave and/or of the secondary wave.

As is known from biophysics studies, cellular membranes have a polarization which is due to the different distribution of ion species between the inside and the outside of the cells. The activation of physiological reactions which involve ion species, in particular enzymatic reactions, modifies the distribution of these ion species and therefore the polarization of the membranes. A measurement of the average polarization value of the cellular membranes of an organism is provided by the body impedance which may be detected by means of a clinical impedance meter.

Advantageously, in accordance with a preferred solution of the invention, the present apparatus allows to accomplish a step (9) for detecting the impedance of an organism irradiated with the abovementioned ELF or ULF variable electromagnetic field $B_c$.

Preferably, the detection step (g) is performed continuously while the organism is irradiated with the variable electromagnetic field $B_c$. In this way it is possible to evaluate in real time the effectiveness of the irradiation on the stimulation of the chosen physiological reactions and therefore establish the duration of the step (b) for generating the electromagnetic field $B_c$.

Once again it is emphasized that the chemical and physical conditions (physiological availability of particular ion species and activation energies) which can be induced in an organism by means of the present method are necessary, but not sufficient, for the activation of particular physiological reactions. In fact, once these chemical and physical conditions have been established, the evolution of these reactions is not automatic, in that it is potentially dependent upon other factors which cannot be controlled and which in particular vary from one organism to another.

With reference to the accompanying figures, the apparatus is denoted in its entirety by 1 and comprises means 20 for generating a variable ELF or ULF electromagnetic field $B_c$ having a non-zero average intensity, and waveform generation means 30 electrically connected to the generation means 20.

Preferably, the generation means 20 comprise one or more Helmholtz coils (not shown) which are of a type known per se and inside which the organism to be irradiated with the abovementioned variable electromagnetic field $B_c$ is arranged. The intensity of this field $B_c$ may vary within the range of $10^{-8}$ to $10^{-4}$ Tesla.

The waveform generation means 30 are connected to the generation means 20 and are able to generate an excitation signal for activating the abovementioned means 20 for generating the variable electromagnetic field $B_c$.

The excitation signal is produced by the superimposition of a main wave and a secondary wave which have different frequencies. In more detail, the main wave has a frequency which is the same as the cyclotron frequency $F_c$ of an ion species to be made physiologically available in the organism (or the harmonic or subharmonic frequency of this cyclotron frequency), while the secondary wave has a frequency within the range of 1 to 100 kHz.

Advantageously, in accordance with the preferred constructional solution shown in Figure 1, the apparatus 1 comprises means for amplifying signals 60 which are electrically arranged between the waveform generation means 30 and the means 20 for generating the electromagnetic field $B_c$.

The apparatus 1 is also provided with at least one magnetometer 40 which is able to detect the intensity of the local static magnetic field $B_0$ in the region of the generation means 20. Preferably, the magnetometer 40 uses Hall-effect sensors, of the type known per se, connected to the Helmholtz coils.

The apparatus 1 is also provided with at least one impedance meter 70, of the type known per se, which can be connected by means of one or more electrodes 71 to the organism while it is irradiated with the abovementioned variable electromagnetic field $B_c$. The impedance meter 70 provides in real time a measurement of the average polarization value of the cellular membranes of the organism during the treatment.

In accordance with the preferred constructional solution shown in Figure 1, the main waveform generation means 30 comprise a first and a second waveform generator 31 and 32 able to generate, respectively, the main wave and the secondary wave. Functionally speaking, the second generator 32 is electrically connected to the first generator ahead of the signal amplification means 60 so as to allow superimposition of the secondary wave on the main wave prior to entry into the generation means 20.

Preferably, the first waveform generator 31 generates a main wave of the pulse type with a non-zero average intensity, while the second waveform generator 32 is a sinusoidal wave generator.

In accordance with another constructional solution, the waveform generation means 30 comprise at least one arbitrary waveform generator, as an alternative to the two generators 31 and 32.

Advantageously, the apparatus 1 is provided, for its operational management, with an electronic processor 50 connected to the magnetometer 40, to the impedance meter 70 and to the waveform generation means 30.

The processor 50 is provided with an internal data base which associates with each type of physiological reaction the ion species to be made physiologically available, the intensity of the MASER fields generated by the expulsion of the ions from the CDF and the impedance value ranges which are considered to be optimum for an organism.

In operational terms, the processor 50 operates, using a given control logic, the waveform generation means 30, defining the frequency and amplitude values of the main wave and secondary wave, which are calculated on the basis of the information contained in the abovementioned database depending on the intensity of the local static magnetic field $B_0$ detected by the means 40.

The processor 50 defines, moreover, the duration time of the treatment depending on the impedance values detected on the organism.

The invention therefore achieves the predefined objects.

Obviously, it may also assume, in its practical embodiment, configurations different from that illustrated above, without thereby departing from the present scope of protection.

**Claims**

1.   Apparatus for making endogenous ions physiologically available in an organism, comprising:

- at least a magnetometer (40) able to detect the intensity of the local static magnetic field ($B_0$) in the space where said organism is placed, in order to define the cyclotron frequency ($F_c$) of an ion species to be made physiologically available in said organism;
- means (20) for generating a variable ELF or ULF electromagnetic field ($B_c$) having a non-zero average intensity, comprising one or more Helmholtz coils, which are able to irradiate said organism with said variable electromagnetic field ($B_c$) ;
- waveform generation means (30), which are electrically connected to said means (20) for generating an electromagnetic field ($B_c$) and are able to generate an excitation signal for activating said generation means (20), **characterized in that**:

said excitation signal is determined by the superimposition of at least one main wave, of the pulse type, having a non-zero average intensity and having a frequency the same as the cyclotron frequency ($F_c$) of said ion species to be made physiologically available in said organism or the harmonic or subharmonic frequency of said cyclotron frequency, and at least one secondary wave, of the sinusoidal type and having a frequency within the range of 1 to 100 kHz corresponding to many cell species resonance frequencies, so that said variable electromagnetic field ($B_c$) is able to interact with the ions of said chosen ion species trapped in CDF present in the intracellular water of the organism so as to cause, by means of resonance, the expulsion of said ions from said CDF, thus making the ions physiologically available inside the cells for subsequent intracellular and/or extracellular metabolic reactions;
said waveform generation means (30) comprising:

- a first waveform generator (31) for generating said main wave; and
- a second waveform generator (32) for generating said secondary wave, which is a sinusoidal wave generator;

and said apparatus further comprises:

- at least one impedance meter (70) which can be connected to said organism while it is irradiated with the abovementioned variable electromagnetic field ($B_c$) ;
- an electronic processor 50, for its operational management, connected to the magnetometer 40, to the impedance meter 70 and to the waveform generation means 30, for defining automatically said waveform.

2. Apparatus for making endogenous ions physiologically available in an organism according to Claim 1, in which said magnetometer (40) is provided with Hall-effect sensors.

3. Apparatus for making endogenous ions physiologically available in an organism according to Claim 1, comprising signal amplification means (60) which electrically connect said waveform generation means (30) to said means (20) for generating an electromagnetic field ($B_c$).

4. Apparatus for making endogenous ions physiologically available in an organism according to Claim 3, in which said second waveform generator (32) is electrically connected to said first waveform generator (31) ahead of said signal amplification means (60) so as to allow superimposition of said secondary wave on said main wave and thus generate said excitation signal.

5. Apparatus for making endogenous ions physiologically available in an organism according to Claim 1, comprising an electronic processor (50), which is connected to said waveform generation means (30) and to said magnetometer (40), said processor (50) defining the frequency and the amplitude of said main wave and said secondary wave depending on the value detected for the intensity of the local static magnetic field ($B_0$) and depending on the ion species to be made physiologically available so as to obtain, at the moment of expulsion of the ions from said CDF, MASER fields of predetermined intensity.

6. Apparatus for making endogenous ions physiologically available in an organism according to Claim 5, in which said processor (50) is connected to said impedance meter (70) and establishes the activation times of said means (20) for generating a variable electromagnetic field ($B_c$) depending on the impedance values detected on said organism.

**Patentansprüche**

1. Apparat, um endogene Ionen in einem Organismus physiologisch verfügbar zu machen, der Folgendes umfasst:

- mindestens ein Magnetometer (40) zur Erfassung der Stärke des lokalen statischen Magnetfelds (B0) in dem Bereich, in dem sich dieser Organismus befindet, um die Zyklotronfrequenz (Fc) einer ionischen Spezies, die im genannten Organismus physiologisch verfügbar gemacht werden soll, zu definieren;
- Mittel (20) zur Erzeugung eines variablen elektromagnetischen ELF- oder ULF-Feldes ($B_c$) mit einer durchschnittlichen Stärke über null, wobei diese Mittel eine oder mehrere Helmholtz-Spulen umfassen und diesen Organismus mit dem genannten variablen elektromagnetischen Feld bestrahlen ($B_c$) können;
- Mittel zur Erzeugung von Wellenformen (30), die elektrisch mit den genannten Mitteln (20) zur Erzeugung eines elektromagnetischen Feldes ($B_c$) verbunden und geeignet sind, ein Erregungssignal zu erzeugen, um die genannten Erzeugungsmittel (20) zu aktivieren, **dadurch gekennzeichnet dass**:

das genannte Erregungssignal sich aus der Überlagerung mindestens einer impulsiven Hauptwelle mit einer durchschnittlichen Stärke über null und einer Frequenz ergibt, die der Zyklotronfrequenz (Fc) dieser in dem genannten Organismus physiologisch verfügbar zu machenden Frequenz oder der Frequenz der Harmonischen oder Subharmonischen der genannten Zyklotronfrequenz entspricht, sowie aus einer sinusförmigen Sekundärwelle mit einer Frequenz im Bereich zwischen 1 und 100 kHz, die vielen Resonanzfrequenzen von Zellspezien entspricht, so dass dieses variable elektromagnetische Feld (Bc) in der Lage ist, mit den Ionen der ausgewählten ionischen Spezies zu interagieren, die in dem im intrazellulären Wasser des Organismus befindlichen CDF eingeschlossen sind, um durch Resonanz die Ausscheidung der Ionen aus den genannten CDF zu bewirken, und somit die Ionen für nachfolgende metabolische intra- und extrazelluläre Reaktionen in den Zellen physiologisch verfügbar zu machen;
wobei die genannten Mittel (30) zur Erzeugung von Wellenformen Folgendes umfassen:

- einen ersten Wellenformengenerator (31) zur Erzeugung der genannten Hauptwelle; und
- einen zweiten Wellenformengenerator (32) zur Erzeugung der genannten Sekundärwelle, der ein Generator von Sinuswellen ist;

und wobei dieser Apparat ferner Folgendes umfasst:

- mindestens einen Widerstandsmesser (70), der mit diesem Organismus bei der Bestrahlung mit dem oben genannten variablen elektromagnetischen Feld ($B_c$) verbunden werden kann;
- einen elektronischen Rechner 50 für dessen Betrieb, der mit dem Magnetometer 40, dem Widerstandsmesser 70 und den Mitteln zur Erzeugung von Wellenformen 30 verbunden ist, um die genannten Wellenformen automatisch zu definieren.

2. Apparat, um endogene Ionen in einem Organismus physiologisch verfügbar zu machen nach Anspruch 1, wobei das genannte Magnetometer (40) mit Hall-Sensoren versehen ist.

3. Apparat, um endogene Ionen in einem Organismus physiologisch verfügbar zu machen nach Anspruch 1, der Mittel zur Signalverstärkung (60) enthält, die diese Mittel (30) zur Erzeugung von Wellenformen mit den Mitteln (20) zur Erzeugung eines Magnetfeldes ($B_c$) elektrisch verbinden.

4. Apparat, um endogene Ionen in einem Organismus physiologisch verfügbar zu machen nach Anspruch 3, wobei dieser zweite Wellenformengenerator (32) mit dem ersten Wellenformengenerator (31) vor den genannten Mitteln zur Signalverstärkung (60) verbunden ist, um die Überlagerung der genannten Hauptwelle von der genannten Sekundärwelle zu gewährleisten und das genannte Erregungssignal zu erzeugen.

5. Apparat, um endogene Ionen in einem Organismus physiologisch verfügbar zu machen nach Anspruch 1, der einen mit den genannten Mitteln (30) zur Erzeugung von Wellenformen und dem genannten Magnetometer (40) verbundenen elektronischen Rechner (50) umfasst, wobei dieser Rechner (50) die Frequenz und die Breite der genannten Hauptwelle und der genannten Sekundärwelle abhängig von dem erfassten Wert für die Stärke des lokalen statischen Magnetfeldes ($B_0$) und der ionischen Spezies, die verfügbar zu machen ist, definiert, um bei der Ausscheidung der Ionen aus diesen CDF MASER-Felder mit einer vorgegebenen Stärke zu erzeugen.

6. Apparat, um endogene Ionen in einem Organismus physiologisch verfügbar zu machen nach Anspruch 5, in dem der genannte Rechner (50) mit dem genannten Widerstandsmesser (70) verbunden ist und die Aktivierungszeiten dieser Mittel (20) zur Erzeugung eines Magnetfeldes ($B_c$) abhängig von den in diesem Organismus erfassten Widerstandswerte definiert.

**Revendications**

1. Appareil pour rendre physiologiquement disponibles des ions endogènes dans un organisme, appareil comprenant :

- un magnétomètre au moins (40) apte à détecter l'intensité du champ magnétique statique local ($B_0$) en correspondance avec l'espace où ledit organisme est placé, pour définir la fréquence de cyclotron (Fc) d'une espèce ionique à rendre physiologiquement disponible dans ledit organisme ;
- des moyens de génération (20) d'un champ électromagnétique ELF ou ULF variable ($B_c$) ayant une intensité moyenne non nulle, comprenant une ou plusieurs bobines de Helmholtz, lesquels moyens sont susceptibles d'irradier ledit organisme par ledit champ électromagnétique variable ($B_c$) ;
- des moyens de génération de formes d'onde (30), lesquels moyens sont électriquement reliés auxdits moyens de génération (20) d'un champ électromagnétique ($B_c$) et ils sont aptes à engendrer un signal d'excitation pour activer lesdits moyens de génération (20), **caractérisé en ce que** :

ledit signal d'excitation est fournis par la superposition d'au moins une onde principale, de type impulsif, ayant une intensité moyenne non nulle et ayant une fréquence égale à la fréquence de cyclotron (Fc) de ladite espèce ionique à rendre physiologiquement disponible dans ledit organisme ou à la fréquence d'harmoniques ou de sous-harmoniques de ladite fréquence de cyclotron, et au moins par une onde secondaire, de type sinusoïdal et ayant une fréquence comprise dans la plage 1 à 100 kHz correspondants à un grand nombres de fréquences de résonance d'espèce cellulaires, de sorte que ledit champ électromagnétique variable (Bc) est susceptibles d'interagir avec les ions de l'espèce ionique choisie piégés en CDF présents dans l'eau intracellulaire de l'organisme de façon à provoquer, par résonance, l'expulsion des ions desdits CDF, en rendant ainsi les ions physiologiquement disponibles au sein des cellules pour ultérieures réactions métaboliques intracellulaires et/ou extracellulaires ;

lesdits moyens de génération de formes d'onde (30) comportant :

- un premier générateur de formes d'onde (31) pour engendrer ladite onde principale ; et
- un deuxième générateur de formes d'ondes (32) pour engendrer ladite onde secondaire, lequel est un générateur d'ondes sinusoïdales ;

et ledit appareil comporte de plus :

- un impédancemètre (70) au moins reliable audit organisme pendant qu'il est irradié par ledit champ électromagnétique variable (Bc) ;
- un calculateur électronique (50), pour sa gestion opérationnelle, connecté au magnétomètre (40), à l'impédancemètre (70) et aux moyens de génération de formes d'onde (30), pour automatiquement définir lesdites formes d'onde.

2. Appareil pour rendre physiologiquement disponibles des ions endogènes dans un organisme selon la revendication 1, dans lequel ledit magnétomètre (40) est pourvu de capteurs à effet Hall.

3. Appareil pour rendre physiologiquement disponibles des ions endogènes dans un organisme selon la revendication 1, comportant de moyens d'amplification de signaux (60), lesquels moyens relient électriquement lesdits moyens de génération de formes d'onde (30) auxdits moyens de génération (20) d'un champ électromagnétique ($B_c$).

4. Appareil pour rendre physiologiquement disponibles des ions endogènes dans un organisme selon la revendication 3, dans lequel ledit deuxième générateur de formes d'onde (32) est électriquement relié audit premier générateur de formes d'onde (31) en amont desdits moyens d'amplification de signaux (60) pour consentir la superposition de ladite onde secondaire sur ladite onde principale et engendrer ainsi ledit signal d'excitation.

5. Appareil pour rendre physiologiquement disponibles des ions endogènes dans un organisme la revendication 1, comportant un calculateur électronique (50), lequel est relié auxdits moyens de génération de formes d'onde (30) et audit magnétomètre (40), ledit calculateur (50) en définissant la fréquence et l'amplitude de ladite onde principale et de ladite onde secondaire en fonction de la valeur détectée d'intensité du champ magnétique statique local ($B_0$) et en fonction de l'espèce ionique à rendre physiologiquement disponible afin d'obtenir, au moment de l'expulsion des ions desdits CDF, des champs MASER d'intensité prédéterminée.

6. Appareil pour rendre physiologiquement disponibles des ions endogènes dans un organisme selon la revendication 5, dans lequel ledit calculateur (50) est relié audit impédancemètre (70) et il établit les temps de déclenchements desdits moyens de génération (20) d'un champ électromagnétique variable ($B_c$) en fonction des valeurs d'impédance détectées sur ledit organisme.

FIG. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4818697 A
- WO 0007664 A
- WO 0076582 A
- EP 0407006 A

**Non-patent literature cited in the description**

- **E. DEL GIUDICE ; M. FLEISCHMANN ; G. PREPARATA ; G. TALPO.** The irrational effects of ELF magnetic fields on an ion system. *Bioelectromagnetics,* 2002, vol. 23, 522-530